# EUROPÄISCHE PATENTSCHRIFT

(11) **EP 1 474 150 B1**
(45) Veröffentlichungstag und Bekanntmachung des Hinweises auf die Patenterteilung: **09.09.2009**
(21) Anmeldenummer: 02748858.4
(22) Anmeldetag: 17.07.2002
(51) Int. Cl.: A61K 31/53, A61P 9/04, A61P 17/06, A61P 15/08, A61P 35/00, A61P 3/10, A61P 27/06, A61P 1/00, A61P 11/12, A61P 15/06, A61P 9/12, A61P 13/10, A61P 13/08, A61P 17/14, A61P 25/16, A61P 23/00, A61P 27/16, A61P 13/12, A61K 31/00

(54) **VERWENDUNG VON 2-ALKOXYPHENYL-SUBSTITUIERTEN IMIDAZOTRIAZINONEN ZUR BEHANDLUNG VON HERZINSUFFIZIENZ**
USE OF 2-ALKOXYPHENYL-SUBSTITUTED IMIDAZOTRIAZINONES FOR THE TREATMENT OF CARDIAC INSUFFICIENCY
UTILISATION D'IMIDAZOTRIAZINONES 2-ALKOXYPHENYL SUBSTITUEES POUR LE TRAITEMENT DE L'INSUFFISANCE CARDIAQUE

(30) Priorität: 23.07.2001 DE 10135815
(43) Veröffentlichungstag der Anmeldung: 10.11.2004
(60) Teilanmeldung: 06017257.4
(73) Patentinhaber: Bayer Schering Pharma AG, 13353 Berlin (DE)
(72) Erfinder: NIEWÖHNER, Ulrich, VERSTORBEN (DE); BISCHOFF, Erwin, 42115 Wuppertal (DE); HANING, Helmut, 42327 Wuppertal (DE); RAHBAR, Afssaneh, 50226 Frechen (DE); BANDEL, Tiemo-Joerg, 46145 Oberhausen (DE); BARTH, Wolfgang, 42349, Wuppertal (DE)
(86) Internationale Anmeldenummer: PCT/EP2002/007959
(87) Internationale Veröffentlichungsnummer: WO 2003/011262

(56) Entgegenhaltungen:
- EP-A- 1 092 719
- EP-A- 1 097 711
- WO-A-94/28902
- WO-A-99/24433
- WO-A-02/064593
- DE-A- 2 811 780
- MIYAHARA MASATOSHI ET AL: "Isoenzymes of cyclic nucleotide phosphodiesterase in the human aorta: Characterization and the effects of E4021." EUROPEAN JOURNAL OF PHARMACOLOGY, Bd. 284, Nr. 1-2, 1995, Seiten 25-33, XP001127423 ISSN: 0014-2999
- BEERS M.H.; BERKOW R.: 'The Merck Manual of Diagnosis and Therapy, Seventeenth Edition', 1999, MERCK RESEARCH LABORATORIES, WHITEHOUSE STATION N.J.

## Beschreibung

Die vorliegende Erfindung betrifft die Verwendung von 2-Alkoxyphenyl-substituierten Imidazotriazinone gemäß Anspruch 1 zur Herstellung von Arzneimitteln zur Behandlung von Herzinsuffizienz.

In der Offenlegungsschrift DE 28 11 780 sind Imidazotriazinone als Bronchodilatoren mit spasmolytischer Aktivität und Hemmaktivität gegen cyclisches Adenosin-monophosphat metabolisierende Phosphodiesterasen (cAMP-PDE's, Nomenklatur nach Beavo: PDE-III und PDE-IV) beschrieben. Eine Hemmwirkung gegen cyclisches Guanosin-monophosphat metabolisierende Phosphodiesterasen (cGMP-PDE's, Nomenklatur nach Beavo und Reifsnyder (Trends in Pharmacol. Sci. 11, 150-155, 1990) PDE-I, PDE-II und PDE-V) ist nicht beschrieben. Es werden keine Verbindungen beansprucht, die eine Sulfonamidgruppe im Arylrest in der 2-Position enthalten. Weiterhin werden Imidazotriazinone in FR 22 13 058, CH 59 46 71, DE 22 55 172, DE 23 64 076 und EP 000 9384 beschrieben, die in der 2-Position keinen substituierten Arylrest besitzen, und ebenfalls als Bronchodilatatoren mit cAMP-PDE inhibitorischer Wirkung beschrieben werden.

In WO 94/28902 werden Pyrazolopyrimidinone beschrieben, die sich für die Behandlung von Impotenz oder Herzinsuffizienz eignen.

In der WO 99/24433 und der WO 99/67244 sind Imidazotriazinone beschrieben, die sich für die Behandlung von Impotenz eignen.

DE2811780 offenbart die Verwendung von 2-Phenyl-substituierten Imidazotriazionen als cAMP-PDE Hemmer zur Behanlung von z.B. Stan-Herzversagen. EP 1097711 offenbart die Verwendungs von cGMP PDES Hemmen, unter anderen Vardenafil, zur Behandlung von pulmonaner Hypertonie.

Zur Zeit sind in der Literatur 11 Phosphodiesterasen mit unterschiedlicher Spezifität gegenüber den cyclischen Nukleotiden cAMP und cGMP beschrieben (Vgl. Fawcett et al., Proc. Nat. Acad. Sci. 97(7), 3072-3077 (2000). Cyclisches Guanosin 3',5'-monophosphat metabolisierende Phosphodiesterasen (cGMP-PDE's) sind die PDE-1, 2, 5, 6, 9, 10, 11. Die erfindungsgemäßen Verbindungen sind potente Inhibitoren der Phosphodiesterase 5. Die differenzierte Expression der Phosphodiesterasen in verschiedenen Zellen, Geweben und Organen, ebenso wie die differenzierte subzelluläre Lokalisation dieser Enzyme, ermöglichen in Verbindung mit den erfindungsgemäßen selektiven Inhibitoren eine selektive Erhöhung der cGMP-Konzentration in spezifischen Zellen, Geweben und Organen und ermöglichen dadurch die Adressierung von verschiedenen von cGMP regulierten Vorgängen. Dies ist besonders zu erwarten wenn unter bestimmten physiologischen Bedingungen die Synthese von cGMP gesteigert ist. Zum Beispiel wird während sexueller Stimulation auf neuronalem Wege Stickstoffmonoxid in den Gefäßen des Corpus Cavernosum freigesetzt und damit die Synthese von cGMP gesteigert. Dies führt zu einer starken Erweiterung der Gefäße, die den Corpus Cavernosum mit Blut versorgen, und damit zur Erektion. Daher sollten Inhibitoren cGMP metabolisierender PDEs besonders für die Behandlung der erektilen Dysfunktion geeignet sein.

Ein Anstieg der cGMP-Konzentration kann zu heilsamen, antiaggregatorischen, antithrombotischen, antiproliferativen, antivasospastischen, vasodilatierenden, natriuretischen und diuretischen Effekten führen und kann die Erregungsleitung im zentralen Nervensystem und damit die Gedächtnisleistung beeinflussen. Es kann die Kurz- oder Langzeitmodulation der vaskulären und kardialen Inotropie, den Herzrhythmus und die kardiale Erregungsleitung beeinflussen (J.C. Stoclet, T. Keravis, N. Komas and C. Lugnier, Exp. Opin. Invest. Drugs (1995), 4 (11), 1081-1100).

Die vorliegende Erfindung betrifft die Verwendung von Verbindungen der

| **Struktur** |
|---|
| 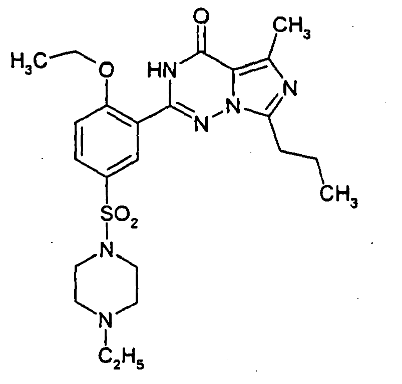 |

und deren Salze, Hydrate und/oder Solvate, zur Herstellung von Arzneimitteln zur Behandlung von Herzinsuffizienz.

Im Rahmen der Erfindung sind physiologisch unbedenkliche Salze bevorzugt. Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen, insbesondere die Salze, können auch als Hydrate vorliegen. Im Rahmen der Erfindung werden unter Hydraten solche Verbindungen verstanden, die im Kristall Wasser enthalten. Solche Verbindungen können ein oder mehrere, typischerweise 1 bis 5, Äquivalente Wasser enthalten. Hydrate lassen sich beispielsweise herstellen, indem man die betreffende Verbindung aus Wasser oder einem wasserhaltigen Lösungsmittel kristallisiert.

Solvate der erfindungsgemäßen Verbindungen sind stöchiomethsche Zusammensetzungen der Verbindungen oder seinen Salzen mit Lösungsmittel.

Die erfindungsgemäßen Verbindungen können gemäß der Beschreibung in der WO 99/24433 hergestellt werden.

Die erfindungsgemäßen Verbindungen hemmen die c-GMP metabolisierende Phosphodiesterase 5. Dies führt zu einem Anstieg von c-GMP. Die differenzierte Expression der Phosphodiesterasen in verschiedenen Zellen, Geweben und Organen, ebenso wie die differenzierte subzelluläre Lokalisation dieser Enzyme, ermöglichen in Verbindung mit den erfindungsgemäßen selektiven Inhibitoren, eine selektive Adressierung der verschiedenen von cGMP regulierten Vorgänge.

Außerdem verstärken die erfindungsgemäßen Verbindungen die Wirkung von Substanzen, wie beispielsweise EDRF (Endothelium derived relaxing factor), ANP (atrial natriuretic peptide), von Nitrovasodilatoren und allen anderen Substanzen, die auf eine andere Art als Phosphodiesterase-Inhibitoren die cGMP-Konzentration erhöhen.

Daher sind die erfindungsgemäßen Verbindungen geeignet zur Prophylaxe und/oder Behandlung von Erkrankungen, bei denen ein Anstieg der cGMP-Konzentration heilsam ist, d.h. Erkrankungen, die im Zusammenhang mit cGMP-regulierten Vorgängen stehen (im Englischen meist einfach als 'cGMPrelated diseases' bezeichnet). Gemäß der vorliegenden Erfindung handelt es sich hierbei um Herzinsuffizienz.

Die Aktivität der Verbindungen der Formel (I) als Inhibitoren der Phosphordiesterasen (PDEs) ist in der WO 99/24433 beschrieben.

Die Wirkstoffe sowie ihre physiologisch unbedenklichen Salze (z.Bsp. Hydrochloride, Maleinate oder Lactate) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 9 Gew.-% der Gesamtmischung vorhanden sein, d.h. in Mengen, die ausreichend sind, um den angegebenen Dosierungsspiekaum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, z.Bsp, perlingual, buccal, intravenös, nasal, rektal oder inhalativ.

Für die Anwendung beim Menschen werden bei oraler Administration Dosierungen von 0,001 bis 50 mg/kg vorzugsweise 0,01 mg/kg - 20 mg/kg sinnvollerweise verabreicht. Bei parenteraler Administration, wie z.B. über Schleimhäute nasal, buccal, inhalativ, ist eine Dosierung von 0,001 mg/kg - 0,5 mg/kg sinnvoll.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der oben genannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die erfindungsgemäßen Verbindungen sind auch zur Anwendung in der Tiermedizin geeignet. Für Anwendungen in der Tiermedizin können die Verbindungen oder ihre nicht toxischen Salze in einer geeigneten Formulierung in Übereinstimmung mit den allgemeinen tiermedizinischen Praxen verabreicht werden. Der Tierarzt kann die Art der Anwendung und die Dosierung nach Art des zu behandelnden Tieres festlegen.

## Patentansprüche

1. Verwendung von 2-Phenyl-substituierten Imidazotriazinonen der Formel 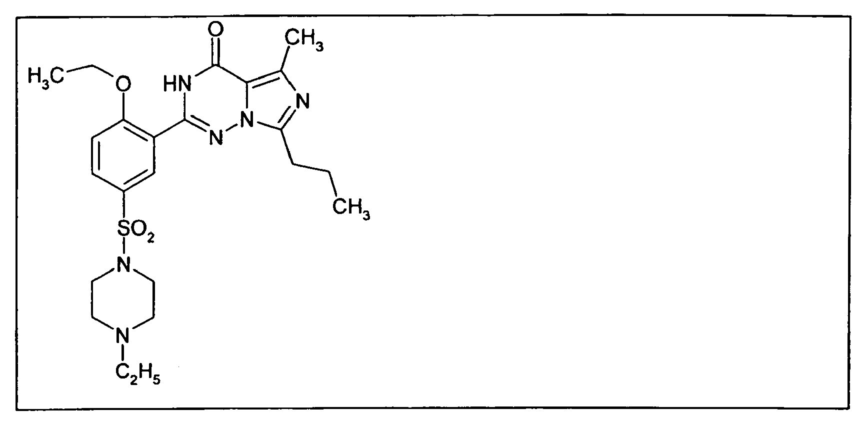 und deren Salze, Hydrate und/oder Solvate,
zur Herstellung von Arzneimitteln zur Behandlung von Herzinsuffizienz.

## Claims

1. Use of 2-phenyl-substituted imidazotriazinones of the formula 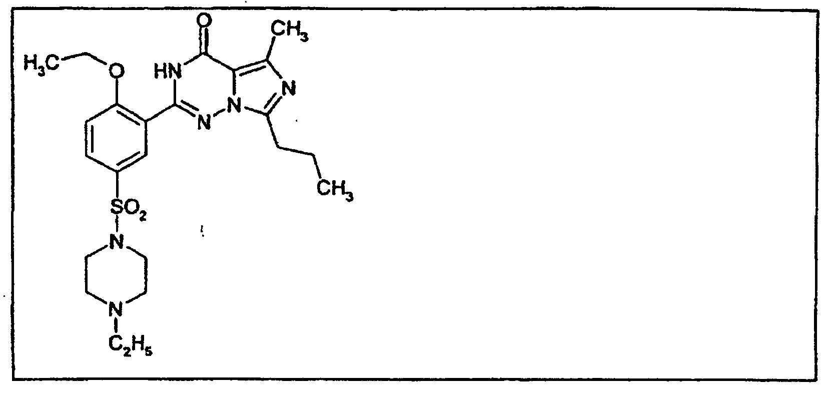 and their salts, hydrates and/or solvates, for the production of medicaments for the treatment of cardiac insufficiency.

## Revendications

1. Utilisation d'imidazotriazinones 2-phényl-substituées de formule 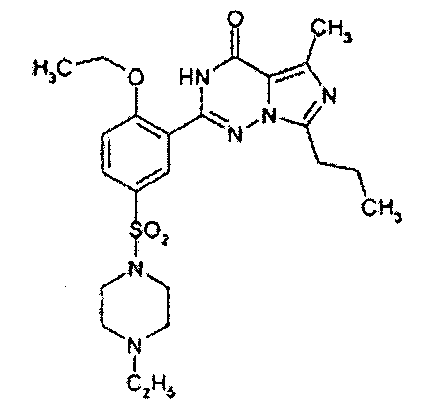 et de leurs sels, leurs hydrates et/ou leurs produits de solvatation pour la préparation de médicaments destinés au traitement de l'insuffisance cardiaque.
